# EUROPEAN PATENT APPLICATION

(11) **EP 2 604 245 A1**
(43) Date of publication of application: **19.06.2013**
(21) Application number: 11368028.4
(22) Date of filing: 16.12.2011
(51) Int. Cl.: A61J 1/20, A61M 39/00, A61M 39/22

(54) **Device for medical purposes**

(71) Applicant: TXCell, 06560 Valbonne (FR)
(72) Inventor: Foussat, Arnaud, 06410 Biot (FR); Belmonte, Nathalie, 06200 Nice (FR); Neveu, Virginie, 25000 Besançon (FR)

(57) **Abstract**

The present invention relates to a device for the preparation of a product to be infused in a subject.

The device comprises:
- at least two containers **(1a)** and **(1b),**
- at least one means for generating a flow **(2a)** able to control the quantity of fluid to flow,
- at least one redirection means **(3a),**

wherein said redirection means selectively allows a fluid communication between said container **(1a),** and said means for generating a flow **(2a)** and between said means for generating a flow **(2a)** and container **(1b).**

## Description

### FIELD OF INVENTION

The present invention relates to a device for medical use, particularly suitable for cell therapy products and their infusion into a subject in need thereof.

### BACKGROUND OF INVENTION

Cell therapy relates to the process of introducing cells into a subject in order to prevent, treat, cure or mitigate a disease or injuries. This new therapy has been made possible by achieving the culture of different human cell strains in the late 90's in the US, and the successful cloning of the first adult mammal from an adult cell.

The ex-vivo preservation of the integrity of the cells used in such a therapy for a certain amount of time is critical to their clinical application. Apart from the improvement of the patient access to cell therapies by increasing the genetic diversity of cells available, ex-vivo preservation allows the cells to be collected in one location, then processed in another location, and finally administered in a third location. The most common technique for long-term cell preservation is the concentration of these cells in a mixture comprising a cryoprotectant such as dimethlysulfoxide (DMSO), followed by a freezing.

After a cryopreservation, cells must be thawed and washed to reduce the cryoprotectant concentration. Cryopreservatives indeed can impair the cell viability after thawing and can be toxic for the patient.

In addition, for some therapies, procedures dictate a minimum and / or a maximum number of cells to be administered to a patient. This can be linked to an absolute value of cells composing the efficient dose for all patients to be administered, or a cell number to be administered to a patient taking into account the body mass or other patient characteristics.

Cell therapy could be considered as a disruptive technology, as it involves new requirements. Until now, such thawing / washing procedures occur in a sterile environment, such as an ISO Class 7 or Class 6 room, and imply the use of biosafety cabinets and specialized facilities. However, these systems in such an environment can be cumbersome for the user, inconvenient, time-consuming to use, and very expensive to build and maintain.

For cryopreserved cell therapy products these three procedures (thawing /washing / preparation of a desired number of cells) are done at clinical sites equipped with specialized facilities. Usually, clinical sites are not equipped with said specialized facilities. Consequently, these three procedures have to be done at another site and then the product is transferred to the clinical site where the patient is to be infused. This implies expensive and complicated logistical resources and is inconvenient and time consuming.

Avoiding transfer of cell therapy products between preparation sites and clinical sites is crucial. Moreover when cells are thawed, delayed injections after thawing can induce low cell stability and cell death leading to inefficient dosing of administration and low therapy efficiency.

Devices relating to thawing / washing procedures are known in the art, such as sterile bags sets of Pall Medical ("cell wash / infusion bag set"). However, even if these devices make the use of certified clean room no longer necessary, they do not provide an easy way - if any - to prepare a desired amount of cells to be administered to the patient.

Thus, there is a need in the art for devices and methods that allow for the convenient, sterile thawing and cryoprotectant reduction of cryopreserved cell therapy products packaged in infusion-ready formulations - these devices allowing also the preparation of a desired amount of cells to be administered.

### SUMMARY

One object of the invention is a device comprising:
- at least two containers (1a) and (1b);
   wherein said container (1a) comprising at least a port (4a) and a port (5a); and wherein said container (1b) comprising at least a port (4b) and a port (5b),
- at least one means for generating a flow (2a),
   wherein said means is able to control the quantity of fluid to flow;
- at least one redirection means (3a);
- optionally at least one means for stopping a flow;
wherein said redirection means (3a) selectively allows a fluid communication between said port (5a) of said container (1a) and said means for generating a flow (2a), and between said means for generating a flow (2a) and said port (4b) of said container (1b);
and wherein said redirection means (3a) prevents a direct fluid communication between said container (1a) and said container (1b).

In one embodiment, said redirection means (3a) is located between said container (1a) and said means for generating a flow (2a), and between said means for generating a flow (2a) and said container (1b).

In another embodiment, said container (1a) further comprises a port (6a), and said container (1b) further comprises a port (6b).

In another embodiment, said device further comprises a means for generating a flow (9) and a redirection means (10) located between the product container (8) and the first container (1a) of the device, wherein:
- said redirection means (10) selectively allows a fluid communication between said product container (8) and said means for generating a flow (9), and between said means for generating a flow (9) and said port (4a) of said container (1a) and;
- wherein said redirection means (10) prevents a direct fluid communication between said product container (8) and said container (1a).

In another embodiment, said device further comprises:
- a third container (1c),
   wherein said container (1c) comprising at least a port (4c) and a port (5c);
- a second means for generating a flow (2b),
   wherein said means for generating a flow is able to control the quantity of fluid to flow;
- a second redirection means (3b);
   wherein said redirection means (3b) selectively allows a fluid communication between said port (5b) of said container (1b) and said means for generating a flow (2b), and between said means for generating a flow (2b) and said port (4c) of said container (1c);
   and wherein said device does not comprise a direct fluid communication between said container (1b) and said container (1c).

In another embodiment, said redirection means (3b) is located between said container (1b) and said means for generating a flow (2b), and between said means for generating a flow (2b) and said container (1c).

In another embodiment, said container (1a) further comprises a port (6a), said container (1b) further comprises a port (6b), and said container (1c) further comprises a port (6c). In another embodiment, said redirection means are multi-way valves, preferably three-way valves, more preferably three-way stopcocks.

In another embodiment, said means for generating a flow are syringes or fluid pumps. In another embodiment, said device is a sterile single-use device.

In another embodiment, at least one of the containers of said device is prefilled with medium before use of the device.

Another object of the invention is the use of the device as described here above for preparing a desired concentration of a product to be infused.

In another embodiment, the product to be infused is a drug, a nutritional complement or a population of cells.

Another object of the invention is the use of the device as described here above for cell therapy, parenteral nutrition or drug administration.

Another object of the invention is a method for preparing a desired concentration of a product comprising the use of the device as described here above, wherein the containers of the device were prefilled with a desired volume of medium, the method comprising the following steps:
- a determined volume of the product is transferred to container (1a) through port (4a);
- the redirection means (3a) is configured so that port (5a) of container (1a) is connected to the means for generating a flow (2a);
- a determined volume of the mixture comprised in the container (1a) is transferred into the means for generating a flow (2a);
- the redirection means (3a) is configured so that the means for generating a flow (2a) is connected to port (4b) of container (1b);
- the content of the means for generating a flow (2a) is then transferred to the container (1b) through port (4b);
thereby the container (1b) contains a ready-to-be infused product at the desired concentration.

Another object of the invention is a method for preparing a desired concentration of a product comprising the use of the device as described here above, wherein the containers of the device were prefilled with a desired volume of medium, the method comprising the following steps:
- a determined volume of the product is transferred to container (1a) through port (4a);
- the redirection means (3a) is configured so that port (5a) of container (1a) is connected to the means for generating a flow (2a);
- a determined volume of the mixture comprised in the container (1a) is transferred into the means for generating a flow (2a);
- the redirection means (3a) is configured so that the means for generating a flow (2a) is connected to port (4b) of container (1b);
- the content of the means for generating a flow (2a) is then transferred to the container (1b) through port (4b);
- the redirection means (3b) is configured so that port (5b) of container (1b) is connected to the means for generating a flow (2b);
- a determined volume of the mixture comprised in the container (1b) is transferred into the means for generating a flow (2b);
- the redirection means (3b) is configured so that the means for generating a flow (2b) is connected to port (4c) of container (1c);
- the content of the means for generating a flow (2b) is then transferred to the container (1c) through port (4c);
thereby the container (1c) contains a ready-to-be infused product at the desired concentration.

### DEFINITIONS

In the present invention, the following terms have the following meanings:
- "Port": refers to an access for a fluid for flowing freely in or out a means containing said port.
- "Valve": refers to a device or part of a device by which the flow of liquid, gas, or loose material may be started, stopped, or regulated, e.g. by a movable part that opens, shuts or partially obstructs one or more ports (e.g., inlet or outlet) or passageways.
- "Polymers": refers to a macromolecule formed by the chemical union of five or more identical combining units called monomers. In most cases, the number of monomer is quite large and often is not precisely known. In synthetic polymers, this number may be controlled to a predetermined extent. Combinations two, three, or four monomers are called, respectively, dimers, trimers, and tetramers, and are known collectively as oligomers. Polymers may be inorganic (e.g., siloxane, sulfur chains, black phosphorus, boron-nitrogen, silicones) or organic (meaning containing carbon). Organic polymers may be natural (e.g., polysaccharides, such as starch, cellulose, pectin, seaweed gums, vegetable gums; polypeptides, such as casein, albumin, globulin, keratin, insulin, DNA; and hydrocarbons), synthetic (e.g, thermoplastics such as unvulcanized elastomers, nylon, polyvinyl chloride, linear polyethylene, polystyrene, polypropylene, polyurethane, acrylate resins; thermosetting such as vulcanized elastomers, crosslinked polyethylene, phenolics, alkyds, polyesters; semisynthetic such as cellulosics, rayon, methylcellulose, cellulose acetate, and modified starches).
- "**Fluid**": refers to a material and/or a combination of materials capable of flowing. For example, a fluid for use in any of the embodiments described herein may include a liquid component and/or a non-liquid component (e.g., gas, solid, semisolid, particulate, colloid) such as in a solution, a suspension, a dispersion or a combination thereof. A fluid as defined here can also comprise a combination of a liquid component and a cellular or tissue-derived material.
- "Direct fluid communication": refers to the free flow of a fluid from one location to another location.

### DETAILED DESCRIPTION

The present invention device solves the problems mentioned in the background of this invention.

First, the device of the invention allows the preparation of different concentrations from a unique product, in a sterile way and at clinical site. Moreover, said concentrations can be adjusted on demand, for example according to a clinical study cohort, the patient weight or the extent of a disease or to any patient or disease characteristics.

Second, for frozen product comprising a cryopreservative, the device of the invention allows a dilution of the cryoprotectant while preparing the desired concentration of the product. Frozen product comprising a cryopreservative is typically a cell therapy product, and reducing the concentration of the cryoprotectant prevents a dramatic impairment of the cells. Moreover, the reduction of the cryopreservative concentration induces a better tolerability for the patient.

Third, for the preparation of a desired concentration of a cell therapy product, this device discards the need for accredited clean rooms as required from regulatory agencies today. This device is thus relatively low-cost, and speeds up the procedures. Fourth, the device of the invention may be connected to an infusion device providing a ready-to-be infused product. According to one embodiment, the infusion device may be an infusion pump or a needle. According to one embodiment, the route of administration for the product to be infused may be enteral or parenteral, preferably parenteral. More preferably, the route of administration for the product to be infused is intravenous, intraosseous, intra-muscular, intracerebral, subcutaneous, intravitreal, intraperitoneal, intravesical, intracardiac, intrathecal, more preferably intravenous.

Last, this convenient device allows easy procedures, and may be used with all kind of fluid product, frozen or not. The method can be carried out in a few minutes, e.g., about 30 minutes or less, preferably about 20 minutes or less, more preferably about 10 minutes or less.

According to the invention, the product to be diluted and infused by using the device of the invention is a drug, a nutritional complement or a population of cells.

In one embodiment, the population of cells may comprise autologous, allogeneic, xenogeneic or recombinant cells.

In one embodiment, the population of cells may comprise somatic cells, stem cells, pluripotent cells, multipotent cells, progenitor cells. In one embodiment, the population of cells may comprise regulatory T cells (Treg) or Type 1 Treg (Tr1) cells.

The cells may be collected from brain, blood, cornea, retina, heart, fat, skin, liver, pancreas, kidney, bladder, spleen, glands, taste buds, nasal cavities, hair follicles, lungs, dental pulp, bone marrow, blood vessels, skeletal muscles, intestines, umbilical cord, placenta, endometrial tissue.

In another embodiment, the drug to be infused may be a small molecule, a chemical compound, a biological compound, DNA, RNA, proteins and peptides, antibodies.

In one embodiment, the product to be diluted and infused contains a cryoprotectant.

The cryoprotectant includes, but is not limited to, DMSO (dimethyl sulfoxide), polyols such as polypropylene glycol, ethylene glycol, 1,2-propanediol (PROH) glycerol, propanediol, inositol, mannitol, sorbitol, 2-methyl-2,4-pentanediol (MPD), propylene glycol, sugars such as glucose, sucrose, maltose, trehalose, lactose, raffinose, stachyose, and dextran. The device of the invention allows a reduction of the cryoprotectant concentration due to one or more dilution(s).

The present invention refers to a device, preferably a single-use sterile device, including means and methods for preparing a desired concentration of a product to be infused in a subject in need thereof.

According to an embodiment, the subject in need thereof is a mammal, for example a human, a cat, a dog, a cow, a monkey, a mouse, a rat or a horse. Preferably, the subject is a human.

The device according to the invention comprises at least two containers, wherein each container may contain a determined amount of a medium before use.

Said containers may be constructed of any material compatible with a biologic fluid, and capable of withstanding a sterilization environment. Typically, these containers may be made from polymeric components. Preferably, these containers are made from plasticized polyvinyl chloride, e.g., PVC plasticized with dioctylphthalate, diethylhexylphthalate, or trioctyltrimellitate. These containers may also be formed from polyolefin, polyurethane, polyester, polycarbonate, polytetrafluoroethylene (PTFE or teflon), glass and stainless steel.

In one embodiment, a container has a capacity of 1, 2, 5, 10, 15, 20, 25, 30, 35, 40, 45, 50, 60, 70, 80, 90, 100, 110, 120, 130, 140, 150, 200, 250, 300, 350, 400, 450 or 500 ml. In one embodiment, the container is a pouch, a bag, a bottle or a vial. In a preferred embodiment, the container is a bag.

According to one embodiment of the invention, each container comprises at least 2 ports. In one embodiment, each container comprises 2 ports. In another embodiment, each container comprises 3 or more ports. In yet another embodiment, the device may comprise containers equipped with 2 ports and containers equipped with 3 ports. In another embodiment, wherein the device comprises containers equipped with 2 ports, at least one port of said containers may be connected to a Y- or T-connector, thereby allowing a total of three or more accesses to said containers.

The device according to the invention further comprises at least one means for generating a flow, and at least one redirection means, arranged such that the content of a container can be transferred in another container. Said means for generating a flow is able to control the quantity of fluid to flow, e.g., said means for generating a flow allows the measurement of the quantity of fluid to flow, and said means for generating a flow can flow a quantity of fluid chosen by the user.

In one embodiment, the redirection means has at least 3 ports, and more preferably 3 ports.

According to one embodiment of the invention, the redirection means has at least 2 distinct configurations, and preferably 2 distinct configurations. In another embodiment, the redirection means has 3 distinct configurations.

In one embodiment, the redirection means allows a fluid communication between at least two of its ports for each configuration. More preferably, the redirection means allows a fluid communication between two of its ports for each configuration. In one embodiment, the redirection means prevents the fluid communication between at least two of its ports, more preferably between two of its ports.

According to one embodiment, the redirection means is a multi-way valve, more preferably, a three-way valve. According to a preferred embodiment, the redirection means is a three-way stopcock.

In one embodiment, the redirection means is composed of one-way valves and Y- or T-connectors. In another embodiment, the redirection means is not composed of one-way valves and Y-connectors.

The redirection means is preferably located between a container and a means for generating a flow.

In one embodiment, the redirection means does not involve gravity. In one embodiment, the redirection means does not involve a power source. In another embodiment, the redirection means involves a power source and may be automated.

According to one embodiment, the redirection means is integrally implemented as one-piece with the means for generating a flow. The one-piece implementation has the advantage of a lower risk of contamination.

In one embodiment, the means for generating a flow is able to control the quantity of fluid to flow.

In one embodiment, the means for generating a flow is a fluid pump. In another embodiment, the means for generating a flow is a syringe-type device, preferably a syringe. In a preferred embodiment, the means for generating a flow is graduated.

In one embodiment, the means for generating a flow may be constructed of any material compatible with a biologic fluid, and capable of withstanding a sterilization environment. Typically, these means for generating a flow may be made from polymeric components. Preferably, these means for generating a flow are made from plasticized polyvinyl chloride, e.g., PVC plasticized with dioctylphthalate, diethylhexylphthalate, or trioctyltrimellitate. These means for generating a flow may also be formed from polyolefin, polyurethane, polyester, polycarbonate, polytetrafluoroethylene (PTFE or teflon), glass and stainless steel.

In one embodiment, the means for generating a flow has at least one port, preferably one port. In one embodiment, at least one port of the means for generating a flow forms a luminal connection with the redirection means, and preferably one port.

In one embodiment, the means for generating a flow can generate a flow in two directions. Said means for generating a flow allows a removal of a desired quantity of fluid from a container forming a luminal connection with said means for generating a flow, and said means for generating a flow allows a release of the desired quantity of fluid to another container forming a luminal connection with said means for generating a flow. The removal may be performed by generating negative pressure (suction, underpressure) with the means for generating a flow. The release may be performed through generation of a pressure difference, i.e. by generating a positive pressure with respect to the surroundings. In a preferred embodiment, the quantity of the removed fluid is exactly the quantity of the released fluid. In one embodiment, said removed fluid is not mixed with another fluid.

In one embodiment, the means for generating a flow is empty before use. In another embodiment, the means for generating a flow is prefilled with a determined quantity of fluid.

In one embodiment, the means for generating a flow is activated manually and does not involve a power source. In another embodiment, the means for generating a flow involves a power source, and may be automated.

In one embodiment, the means for generating a flow does not involve gravity.

In one embodiment, the means for generating a flow has a capacity of 0.1, 0.2, 0.5, 1, 2, 5, 10, 15, 20, 25, 30, 35, 40, 45, 50, 60, 70, 80, 90, 100, 110, 120, 130, 140, 150, 200 or 250 ml.

In one embodiment, the means for generating a flow allows a volume measurement with a precision below 5 ml, preferably below 2 ml, more preferably below 1 ml, even more preferably below 0.5 ml, even more preferably below 0.2 ml or 0.1 ml.

In one embodiment, the container may comprise at least one port, said port being preferably sterile.

In one embodiment, wherein each container comprises two ports, each port of a container is connected to a redirection means, except for the first container whose first port is for example for the connection to another container containing the product to be diluted, and for the last container whose second port is for example for the connection to an infusion device. For example, each port may be a spike, a MPC or a luer lock port. The first port of the first container may be for example connected to a spike.

The second port of the last container may be for example a spike port. The second port of the last container may be connected to a spike.

In another embodiment, wherein each container comprises more than two ports, at least one of the ports is designed for the filling of said container by a medium. In this embodiment, said ports may be spikes, MPC or luer lock ports.

In one embodiment, the first container of the device is designed to receive the product to be diluted. Thus, a first dilution occurs when the first container is prefilled with a determined amount of a medium.

In one embodiment, the medium used to dilute the product includes but is not limited to human serum albumin (HSA), dextrose, Plasma-Lyte (Baxter), Normasol (Abbott), microspheres, plasma, saline solutions.

In one embodiment, the medium used to dilute a product is an HSA-based medium.

According to one embodiment of the invention, the first container is connected to a perforator connector such as a spike. Said connector may be useful to connect the first container to another container containing the product to be diluted before infusion, for example a pouch containing cells for cell therapy.

After a partial thawing of a product packaged in a cryobag for example, another advantage of this device is to allow said product to be thawed in a sterile and a safer way for the cells. In this embodiment, said cryobag may be for example first immersed in a warm bath in order to partially thaw the product inside. The first container is then connected to said cryobag, and a transfer of a medium from said first container to said cryobag allows the total thawing of the product. This process has the further advantage to rapidly dilute the cryopreservative in the medium after the thawing, preventing thus a dramatic cells impairment.

In one embodiment, a means for generating a flow and a redirection means may be located between the container containing the product to be diluted and the first container of the device of the invention, connected in the same way than a means for generating a flow and a redirection means between two containers. This embodiment allows the transfer to the first container of the device of a determined quantity/volume of the product to be diluted. In this embodiment, the redirection means located between the product container and the first container of the device may be connected to a spike to make the connection with the product container.

In another embodiment, the means for generating a flow is prefilled with a determined quantity of a medium or of another product to be diluted such as for example cell population, drug, and nutritional complement. This embodiment allows the mixture of two products to be diluted and their transfer to the first container of the device.

In another embodiment, the content of the product container is entirely transferred in the first container of the invention.

After the transfer of fluid from the first container to a second container, another dilution occurs when said second container is prefilled with a determined amount of a medium.

In one embodiment, the last container contains the desired concentration of the product to be infused. In one embodiment, said container comprises a means, such as a spike port or a spike, to connect an infusion device. In this embodiment, after the successive dilutions said container contains a ready-to-be-infused product.

In one embodiment, the last container may be disconnected in a sterile way from the last redirection means. Said disconnection may be performed for example by a thermal welder or an ultrasonic device. In this embodiment, the last container containing the product to be infused is easily mobile.

In one embodiment, the device is designed such that the fluid communication between two containers is controlled by an assembly composed of a means for generating a flow and a redirection means, said means for generating a flow being able to control the quantity of fluid to flow. In one embodiment, the device or the redirection means prevents a direct fluid communication between two containers.

In one embodiment, the different elements of the device of the invention are connected in part by conduit means, allowing the fluid communication.

In one embodiment, the conduit means may be made of any type of medical grade, sterilizable, durable tubing suitable for transporting the fluid or gas in use. For example, the conduit means can be flexible or rigid plastic.

In one embodiment, conduit means are fluid lines.

In one embodiment, each connection between two elements of the device may comprise a fitting, preferably a sterile fitting.

In one embodiment, the fluid transfer from a first container to a second container is carried out by two steps. The first step is the removal of the desired quantity of fluid from the first container by the means for generating a flow via the redirection means. The second step is the release of the removed fluid from the means for generating a flow to the second container via the redirection means. Between the aspiration step and the expulsion step, the redirection means is reconfigured to redirect the flow of the fluid.

In one embodiment of the invention, each redirection means is connected to two containers and one means for generating a flow. According to this embodiment, the redirection means can selectively establish a fluid communication between one container and one means for generating a flow, and between said means for generating a flow and another container. In a further embodiment, each redirection means prevents a fluid communication between two containers connected by said redirection means.

In one embodiment, the device of the invention comprises at least two containers. In one embodiment, the device of the invention comprises two containers. In said embodiment, the two containers are connected to one means for generating a flow via one redirection means.

In another embodiment, the device of the invention comprises three or more containers. In said embodiment, the three or more containers are connected to two or more means for generating a flow via two or more redirection means, wherein two containers are connected to one means for generating a flow via one redirection means.

In one embodiment, the device of the invention may further comprise at least one means for stopping a flow, located preferably on / in conduits connected to a container port or on / in a port of a container. Means for stopping a flow may be stoppers, 2-way valves, pinches or clamps, and are preferably used as security means to prevent an undesired flow of fluid.

In one embodiment, wherein no means for generating a flow and no redirection means is located between the container containing the product to be diluted and the first container of the invention, the device comprises two means for stopping a flow: the first one is located between the product container and the first container of the device, and the second one is located between the first container of the device and the redirection means connected to said container. In this embodiment, the device comprises preferably two containers.

In another embodiment, wherein no means for generating a flow and no redirection means is located between the container containing the product to be diluted and the first container of the invention, the device comprises four means for stopping a flow: the first one is located between the product container and the first container of the device, the second one is located between the first container of the device and the redirection means connected to said container, the third one is located between said redirection means and the second container of the device and the fourth one is located between the second container of the device and the other redirection means connected to said second container. In this embodiment, the device comprises preferably three containers.

In one embodiment, wherein a means for generating a flow and a redirection means are located between the container containing the product to be diluted and the first container of the invention, the device comprises three means for stopping a flow: the first one is located between the product container and the first redirection means, the second one is located between said first redirection means and the first container of the device, and the third one is located between the first container of the device and the other redirection means connected to said container. In this embodiment, the device comprises preferably two containers.

In another embodiment, wherein a means for generating a flow and a redirection means are located between the container containing the product to be diluted and the first container of the invention, the device comprises five means for stopping a flow: the first one is located between the product container and the first redirection means, the second one is located between said first redirection means and the first container of the device, the third one is located between the first container of the device and the other redirection means connected to said container, the fourth one is located between said redirection means and the second container of the device and the fifth one is located between the second container of the device and the other redirection means connected to said second container. In this embodiment, the device comprises preferably three containers.

In yet another embodiment, the device comprises an additional means for stopping a flow located between the last container of the device and the redirection means connected to said container, so that the fluid communication between the last container of the device and the redirection means connected to said container may be stopped before disconnecting the last container from the rest of the device. In this embodiment, the means for stopping a flow may be located just before a sterile welding. In this embodiment, the device comprises preferably three means for stopping a flow if said device comprises two containers and one means for generating a flow, or preferably four means for stopping a flow if said device comprises two containers and two means for generating a flow, or preferably five means for stopping a flow if said device comprises three containers and two means for generating a flow, or preferably six means for generating a flow if said device comprises three containers and three means for generating a flow.

In one embodiment, the means for stopping a flow are mobile.

In one embodiment, the device of the invention is sterilizable, for example via heat (such as autoclaving, flaming, incineration, boiling, tindalization), ionizing or non-ionizing radiation (such as UV, X-rays, gamma-rays, electron beams, subatomic particles), ultrasound, high pressure, sterile filtration or via a chemical process (such as ethylene oxide, ozone, chlorine bleach, glutaraldehyde and formaldehyde, phthalaldehyde, hydrogen peroxide, peracetic acid, silver).

In one embodiment, the device contains no seeding means.

The invention also relates to a method for preparing a desired concentration of a product to be infused in a subject in need thereof.

In one embodiment wherein the device comprises two containers, the first and the second container are filled with a determined amount of medium. In another embodiment, the containers are pre-filled before use.

A determined amount of a product to be diluted and infused is then flowed via the first port of the first container.

A first dilution occurs in the first container, according to the concentration the user is willing to reach.

The user configures the redirection means such that the redirection means establishes a luminal connection between the means for generating a flow and the first container. The user transfers a determined quantity of the mixture contained in the first container to the means for generating a flow via said redirection means. Then said redirection means is configured such that said redirection means establishes a luminal connection between said means for generating a flow and the second container. Finally the user transfers the volume contained in the means for generating a flow to the second container via the redirection means. A second dilution occurs in the second container, according to the concentration the user is willing to reach.

In one embodiment, dead volumes exist in the conduit means. In this embodiment, these volumes are known by the user and the transfer is adjusted if needed so that finally, the right volume of fluid to transfer is reached.

If the device comprises more than two containers, other dilutions can be made. Once the last dilution has occurred in the last container, the last container thereby contains the ready-to-be infused product at the desired concentration.

In one embodiment, wherein the device comprises one or more means for stopping a flow, it is possible to use them in order to stop the flow back after any transfer of fluid. According to an embodiment, the device is for use in cell therapy.

In one embodiment, the device of the invention may be used for preparing a desired concentration of the product to be infused, said desired concentration being 2x11⁻² cells/ml.

In said embodiment, the device of the invention comprises three containers and the product to be infused is a 5 ml population of cells frozen at the concentration of 5x10⁶ cells/ml.

In this embodiment, the method for preparing the desired concentration of cells to be infused comprises:
- The first container of the device is filled with 245 ml of a medium, the second container with 45 ml of a medium and the third container with 98 ml of a medium.
- The initial product is entirely added to the first container, leading to a first dilution. A concentration of 1x10⁵ cells/ml is then reached in the first container.
- The first redirection means is configured so that said redirection means forms a luminal connection between the first container and the first means for generating a flow.
- 5 ml of the mixture from the first container is transferred in the first means for generating a flow.
- The first redirection means is reconfigured such that said redirection means allows a fluid communication between the first means for generating a flow and the second container.
- The content of said means for generating a flow is then transferred to the second container. A second dilution occurs in the second container leading to a concentration of 1x10⁴ cells/ml.
- The second redirection means is configured so that said redirection means forms a luminal connection between the second container and the second means for generating a flow.
- 2 ml of the mixture of the second container is then transferred in the second means for generating a flow.
- The second redirection means is reconfigured such that said redirection means allows a fluid communication between the second means for generating a flow and the third container.
- The content of said means for generating a flow is finally transferred to the third container. A third dilution occurs in the third container leading to a concentration of 2x10² cells/ml, in a volume ready to be infused.

Having a 100 ml volume at a concentration of 2x10² cells/ml in the third container, two injections of 50 ml containing 10⁴ cells are possible. Moreover, if the initial product comprises a cryopreservative, said cryopreservative is diluted by a factor 25000.

In another embodiment, the device of the invention may be used for preparing a desired concentration of the product to be infused, said desired concentration being 2x10³ cells/ml.

In said embodiment, the device of the invention comprises two containers and the product to be infused is a 5 ml population of cells frozen at the concentration of 5x10⁶ cells/ml.

In this embodiment, the method for preparing the desired concentration of cells to be infused comprises:
- The first container of the device is filled with 245 ml of a medium and the second container with 98 ml of a medium.
- The initial product is entirely added to the first container, leading to a first dilution. A concentration of 1x10⁵ cells/ml is then reached in the first container.
- The first redirection means is configured so that said redirection means forms a luminal connection between the first container and the first means for generating a flow.
- 2 ml of the mixture from the first container is transferred in the first means for generating a flow.
- The first redirection means is reconfigured such that said redirection means allows a fluid communication between the first means for generating a flow and the second container.
- The content of said means for generating a flow is then transferred to the second container. A second dilution occurs in the second container leading to a concentration of 2x10³ cells/ml, in a volume ready-to-be infused.

Having a 100 ml volume at a concentration of 2x10³ cells/ml in the second container, two injections of 50 ml containing 10⁵ cells are possible. Moreover, if the initial product comprises a cryopreservative, said cryopreservative is diluted by a factor 2500.

In another embodiment, the device of the invention may be used for preparing a desired concentration of the product to be infused, said desired concentration being 2x10⁴ cells/ml.

In said embodiment, the device of the invention comprises two containers and the product to be infused is a 5 ml population of cells frozen at the concentration of 5x10⁶ cells/ml.

In this embodiment, the method for preparing the desired concentration of cells to be infused comprises:
- The first container of the device is filled with 45 ml of a medium and the second container with 96 ml of a medium.
- The initial product is entirely added to the first container, leading to a first dilution. A concentration of 5x10⁵ cells/ml is then reached in the first container.
- The first redirection means is configured so that said redirection means forms a luminal connection between the first container and the first means for generating a flow.
- 4 ml of the mixture from the first container is transferred in the first means for generating a flow.
- The first redirection means is reconfigured such that said redirection means allows a fluid communication between the first means for generating a flow and the second container.
- The content of said means for generating a flow is then transferred to the second container. A second dilution occurs in the second container leading to a concentration of 2x10⁴ cells/ml, in a volume ready-to-be infused.

Having a 100 ml volume at a concentration of 2x10⁴ cells/ml in the second container, two injections of 50 ml containing 10⁶ cells are possible. Moreover, if the initial product comprises a cryopreservative, said cryopreservative is diluted by a factor 250.

In another embodiment, the device of the invention may be used for preparing a desired concentration of the product to be infused, said desired concentration being 2x10⁵ cells/ml.

In said embodiment, the device of the invention comprises two containers and the product to be infused is a 5 ml population of cells frozen at the concentration of 5x10⁶ cells/ml.

In this embodiment, the method for preparing the desired concentration of cells to be infused comprises:
- The first container of the device is filled with 20 ml of a medium and the second container with 80 ml of a medium.
- The initial product is entirely added to the first container, leading to a first dilution. A concentration of 1x10⁶ cells/ml is then reached in the first container.
- The first redirection means is configured so that said redirection means forms a luminal connection between the first container and the first means for generating a flow.
- 20 ml of the mixture from the first container is transferred in the first means for generating a flow.
- The first redirection means is reconfigured such that said redirection means allows a fluid communication between the first means for generating a flow and the second container.
- The content of said means for generating a flow is then transferred to the second container. A second dilution occurs in the second container leading to a concentration of 2x10⁵ cells/ml, in a volume ready-to-be infused.

Having a 100 ml volume at a concentration of 2x10⁵ cells/ml in the second container, two injections of 50 ml containing 10⁷ cells are possible. Moreover, if the initial product comprises a cryopreservative, said cryopreservative is diluted by a factor 25.

### BRIEF DESCRIPTION OF THE DRAWINGS

The figures show:
**Figure 1****:** a device comprising two containers wherein each container comprises two ports. A spike is connected to the first container of the invention.
**Figure 2****:** a device comprising two containers, wherein each container comprises three ports. The device further comprises a redirection means and a means for generating a flow, located between the first container and a container containing a product.
**Figure 3****:** a device comprising three containers, wherein each container comprises two ports. The first container is connected to a spike.
**Figure 4****:** a device comprising three containers, wherein each container comprises three ports. A container containing a product is connected to the first container of the device via a spike. The device further comprises four means for stopping a flow.

### FIGURE 1

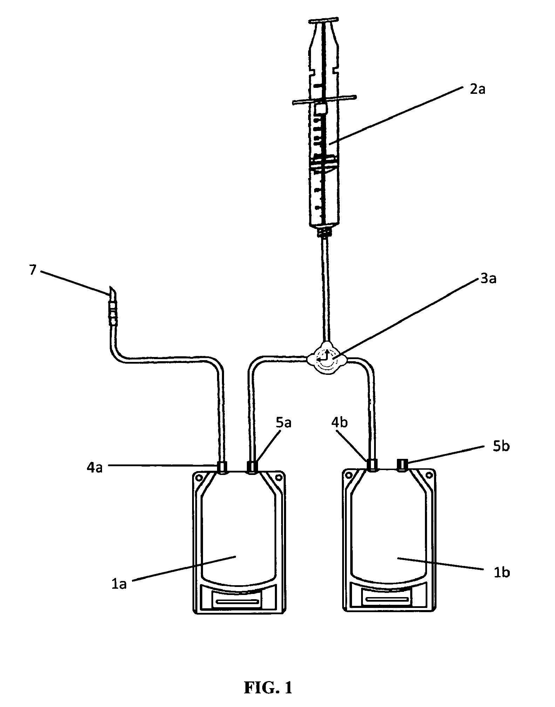

Figure 1 shows two containers **1a** and **1b** in the form of a bag, one redirection means **3a** (here a three-way stopcock), and one means for generating a flow **2a** (here a syringe). Container **1a** comprises two ports **4a** and **5a.** Each container **1a** and **1b** may be prefilled with a determined quantity of a medium. Container **1b** comprises two ports **4b** and **5b.** The redirection means **3a** allows a fluid communication selectively between port **5a** of container **1a** and the means for generating a flow **2a** and between the means for generating a flow **2a** and port **4b** of container **1b.** No direct fluid communication is made possible between container **1a** and container **1b.**

The container **1a** may be connected to another container containing the product to be diluted via port **4a,** and for example via a spike 7.

The container **1b** may be connected to an infusion device via port **5b,** which can be for example a spike port.

The different elements of the device are connected by fluid lines, allowing the fluid communication.

### FIGURE 2

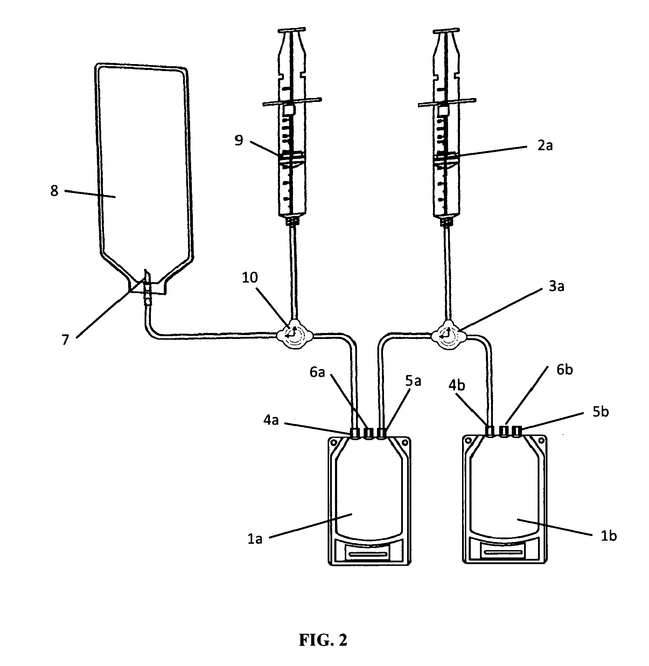

Figure 2 shows two containers **1a** and **1b** in the form of a bag, two redirection means **3a** and **10** (here three-way stopcocks), and two means for generating a flow **2a** and **9** (here syringes). The product to be diluted is in a third container **8.** Each container **1a** and **1b** may be prefilled with a determined quantity of a medium. Container **1a** comprises three ports **4a, 5a** and **6a.** Container **1b** comprises three ports **4b, 5b** and **6b.** The redirection means **10** allows a fluid communication selectively between container **8** and the means for generating a flow **9** and between the means for generating a flow **9** and port **4a** of container **1a.** No direct communication is made possible between container **8** and container **1a.** The redirection means **10** may be connected to the container **8** via a spike 7. The redirection means **3a** allows a fluid communication selectively between port **5a** of container **1a** and the means for generating a flow **2a** and between the means for generating a flow **2a** and port **4b** of container **1b.** No direct fluid communication is made possible between container **1a** and container **1b.**

Container **1b** may be connected to an infusion device via port **5b,** which can be for example a spike port.

Container **1a** and container **1b** may be filled with medium respectively with port **6a** and port **6b.**

The different elements of the device are connected by fluid lines, allowing the fluid communication.

### FIGURE 3

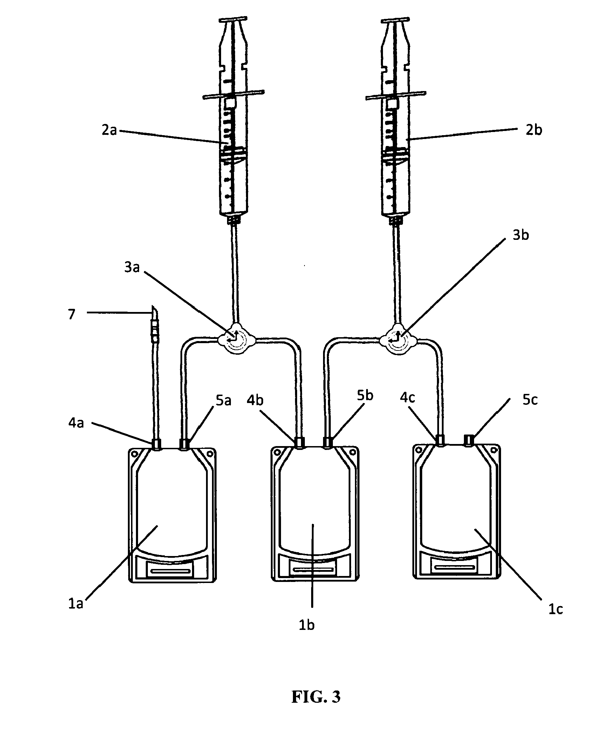

Figure 3 shows three containers **1a, 1b** and **1c** in the form of a bag, two redirection means **3a** and **3b** (here three-way stopcocks), and two means for generating a flow **2a** and **2b** (here syringes). Each container **1a, 1b** and **1c** may be prefilled with a determined quantity of a medium. Container **1a** comprises two ports **4a** and **5a.** Container **1b** comprises two ports **4b** and **5b.** Container **1c** comprises two ports **4c** and **5c.** The redirection means **3a** allows a fluid communication selectively between port **5a** of container **1a** and the means for generating a flow **2a** and between the means for generating a flow 2a and port 4b of container **1b**. The redirection means 3b allows a fluid communication selectively between port 5b of container **1b** and the means for generating a flow 2b and between the means for generating a flow 2b and port 4c of container **1c**. No direct fluid communication is made possible between container 1a and container 1b and between container **1b** and container **1c**.

The container 1a may be connected to another container containing the product to be diluted via port 4a, and for example via a spike 7.

The container **1c** may be connected to an infusion device via port 5c, which can be for example a spike port.

The different elements of the device are connected by fluid lines, allowing the fluid communication.

### FIGURE 4

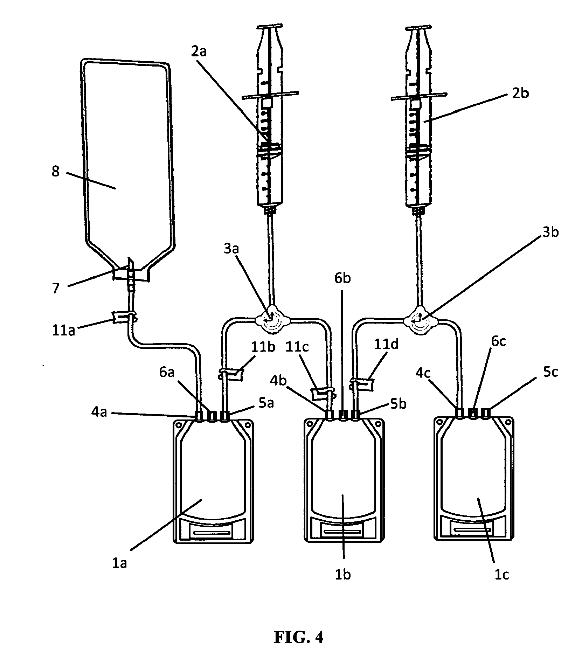

Figure 4 shows three containers 1a, **1b** and **1c** in the form of a bag, two redirection means 3a and 3b (here three-way stopcocks), and two means for generating a flow 2a and 2b (here syringes). The device may further comprise four means for stopping a flow **11a, 11b, 11c**, and **11d**, for example clamps. The product to be diluted is in a fourth container 8. Each container **1a**, **1b** and **1c** may be prefilled with a determined quantity of a medium. Container 1a comprises three ports 4a, 5a and 6a. Container **1b** comprises three ports 4b, 5b and 6b. Container **1c** comprises three ports **4c**, 5c and 6c. The redirection means 3a allows a fluid communication selectively between port 5a of container **1a** and the means for generating a flow **2a** and between the means for generating a flow **2a** and port **4b** of container **1b.** The redirection means **3b** allows a fluid communication selectively between port **5b** of container **1b** and the means for generating a flow **2b** and between the means for generating a flow **2b** and port **4c** of container **1c.** No direct fluid communication is made possible between container **1a** and container **1b,** and between container **1b** and container **1c.**

The container **1a** may be connected to another container containing the product to be diluted via port **4a,** and for example via a spike 7.

The container **1c** may be connected to an infusion device via port **5c,** which can be for example a spike port.

Container **1a,** container **1b** and container **1c** may be filled with medium respectively with port **6a,** port **6b** and port **6c.**

The different elements of the device are connected by fluid lines, allowing the fluid communication.

### REFERENCE NUMBER

- **1**: Container
- **2**: Means for generating a flow
- **3**: Redirection means
- **4**: Port
- **5**: Port
- **6**: Port
- **7**: Spike
- **8**: Container containing a product to be diluted
- **9**: Means for generating a flow
- **10**: Redirection means
- **11**: Means for stopping a flow

### EXAMPLE 1

The product is a 5 ml population of Tr1 cells frozen at the concentration of 5x10⁶ cells/ml. The desired concentration of Tr1 cells to be obtained for infusion is 2x10³ cells/ml.

The device used in these conditions comprises two containers such as shown in Figure 1: the first container **1a** is prefilled with 245 ml of an HSA-based medium and the second container **1b** is prefilled with 98 ml of the medium.

The method for preparing said concentration of cells is the following:
- The spike 7 connected to port **4a** of container **1a** is plugged in the container containing the population of cells.
- The Tr1 cells, previously thawed, are entirely transferred in the first container **1a** through its port **4a.** A concentration of 1x10⁵ cells/ml is reached in the first container **1a.**
- The three-way stopcock **3a** is configured such that it allows a fluid communication between port **5a** of container **1a** and syringe **2a.**
- 2 ml of the mixture contained in container **1a** is sucked up and reaches the syringe **2a** via the three-way stopcock **3a.**
- When the syringe **2a** is filled, the three-way cock **3a** is switched to allow a fluid communication between the syringe **2a** and port **4b** of container **1b.**
- The syringe **2a** is then emptied by applying pressure and the mixture flows in the second container **1b** via the three-way stopcock **3a.** A second dilution occurs in the second container and a concentration of 2x10³ cells/ml is reached.
- The desired concentration of the product is reached.

The container **1b** may be disconnected from the three-way stopcock with a thermal welder.

Then, the container **1b** is carried to the patient to be infused and an infusion device may be connected to port **5b** of container **1b** for carrying out the infusion.

Having a 100 ml volume at a concentration of 2x10³ cells/ml in the second container **1b,** two injections of 50 ml containing 10⁵ cells are possible. Moreover, if the initial product comprises a cryoprotectant, said cryoprotectant is diluted by a factor 2500.

### EXAMPLE 2

The product is a 10 ml population of Tr1 cells frozen at the concentration of 5x10⁶ cells/ml. The desired concentration of Tr1 cells to be obtained for infusion is 2x10⁴ cells/ml.

The device used in these conditions comprises two containers such as shown in Figure 2: the first container **1a** was filled through its port **6a** with 45 ml of HSA medium and the second container **1b** was filled through its port **6b** with 96 ml of HSA medium.

The method for preparing said concentration of cells is the following:
- The spike **7** connected to the three-way stopcock **10** is plugged in the container **8**.
- The three-way stopcock **10** is configured such that it allows a fluid communication between container **8** and the syringe **9.**
- 5 ml of the Tr1 cells, previously thawed and contained in the container **8,** is sucked up and reaches the syringe **9** via the three-way stopcock **10.**
- When the syringe **9** is filled, the three-way stopcock **10** is switched to allow a fluid communication between the syringe **9** and port **4a** of container **1a.**
- The syringe **9** is emptied by applying a pressure and the Tr1 cells are transferred in the first container **1a** through its port **4a.** A concentration of 5x10⁵ cells/ml is reached in the first container **1a.**
- The three-way stopcock **3a** is configured such that it allows a fluid communication between port **5a** of container **1a** and said syringe **2a.**
- 4 ml of the mixture contained in container **1a** is sucked up and reaches the syringe **2a** via the three-way stopcock **3a.**
- When the syringe **2a** is filled, the three-way stopcock **3a** is switched to allow a fluid communication between the syringe **2a** and port **4b** of container **1b.**
- The syringe **2a** is then emptied by applying pressure and the mixture flows in the second container **1b** via the three-way stopcock **3a.** A second dilution occurs in the second container and a concentration of 2x10⁴ cells/ml is reached.
- The desired concentration of the product is reached.

The container **1b** may be disconnected from the three-way stopcock with a thermal welder.

Then, the container **1b** is carried to the patient to be infused and an infusion device may be connected to port **5b** of container **1b** for carrying out the infusion.

Having a 100 ml volume at a concentration of 2x10⁴ cells/ml in the second container **1b,** two injections of 50 ml containing 10⁶ cells are possible. Moreover, if the initial product comprises a cryoprotectant, said cryoprotectant is diluted by a factor 250.

### EXAMPLE 3

The product is a 5 ml population of Tr1 cells frozen at the concentration of 5x10⁶ cells/ml. The desired concentration of Tr1 cells to be obtained for infusion is 2x10² cells/ml.

The device used in these conditions comprises three containers such as shown in Figure 4.

The method for preparing said concentration of cells is the following:
- The first container **1a** is filled through its port **6a** with 245 ml of a HSA medium.
- The second container **1b** is filled through its port **6b** with 45 ml of the medium.
- The third container **1c** is filled through its port **6c** with 98 ml of the medium.
- The spike **7** connected to port **4a** of container **1a** is plugged in the container **8** containing the population of cells.
- The clamp **11a** is removed so that a fluid can flow through the port **4a** of the container **1a.**
- The Tr1 cells, previously thawed, are entirely transferred in the first container **1a** through its port **4a.** A concentration of 1x10⁵ cells/ml is reached in the first container **1a.**
- The clamp **11a** is placed in order to prevent a fluid to flow through the port **4a** of container **1a.**
- The three-way stopcock **3a** is configured such that it allows a fluid communication between port **5a** of container **1a** and syringe **2a.**
- The clamp **11b** is removed so that a fluid can flow through the port **5a** of the container **1a.**
- 5 ml of the mixture contained in container **1a** is sucked up and reaches the syringe **2a** via the three-way stopcock **3a.**
- The clamp **11b** is placed in order to prevent a fluid to flow through the port **5a** of the container **1a.**
- When the syringe **2a** is filled, the three-way cock **3a** is switched to allow a fluid communication between the syringe **2a** and port **4b** of container **1b.**
- The clamp **11c** is removed so that a fluid can flow through the port **4b** of the container **1b.**
- The syringe **2a** is then emptied by applying pressure and the mixture flows in the second container **1b** via the three-way stopcock **3a.** A second dilution occurs in the second container and a concentration of 1x10⁴ cells/ml is reached.
- The clamp **11c** is placed is order to prevent a fluid to flow through the port **4b** of the container **1b.**
- The second three-way cock **3b** is switched to form a luminal connection between port **5b** of the second container **1b** and the syringe **2b.**
- The clamp **11d** is removed so that a fluid can flow through the port **5b** of the container **1b.**
- 2 ml of the mixture of the second container **1b** is sucked up and reaches the syringe **2b** via the three-way cock **3b.**
- The clamp **11d** is placed in order to prevent a fluid to flow through the port **5b** of the container **1b.**
- The second three-way cock **3b** is switched to allow a fluid communication between the second syringe **2b** and port **4c** of the third container **1c.**
- The syringe **2b** is then emptied by applying pressure and the mixture flows to the third container **1c** via the three-way cock **3b.** A third dilution occurs in the third container and a concentration of 2x10² cells/ml is reached in the third container.
- The desired concentration of the product is reached.

The container **1c** may be disconnected from the three-way stopcock with a thermal welder.

Then, the container **1c** is carried to the patient to be infused and an infusion device may be connected to port **5c** of container **1c** for carrying out the infusion.

Having a 100 ml volume at a concentration of 2x10² cells/ml in the third container **1c**, two injections of 50 ml containing 10⁴ cells are possible. Moreover, if the initial product comprises a cryoprotectant, said cryoprotectant is diluted by a factor 25000.

## Claims

1. A device comprising:
- at least two containers (1a) and (1b);
wherein said container (1a) comprising at least a port (4a) and a port (5a); and wherein said container (1b) comprising at least a port (4b) and a port (5b),
- at least one means for generating a flow (2a),
wherein said means is able to control the quantity of fluid to flow;
- at least one redirection means (3a);
- optionally at least one means for stopping a flow;
wherein said redirection means (3a) selectively allows a fluid communication between said port (5a) of said container (1a) and said means for generating a flow (2a), and between said means for generating a flow (2a) and said port (4b) of said container (1b);
and wherein said redirection means (3a) prevents a direct fluid communication between said container (1a) and said container (1b).

2. The device according to claim **1,** wherein said redirection means (3a) is located between said container (1a) and said means for generating a flow (2a), and between said means for generating a flow (2a) and said container (1b).

3. The device according to claim **1** or claim **2,** wherein said container (1a) further comprises a port (6a), and said container (1b) further comprises a port (6b).

4. The device according to any one of claims **1 to 3,** wherein said device further comprises a means for generating a flow (9) and a redirection means (10) located between the product container (8) and the first container (1a) of the device, wherein:
- said redirection means (10) selectively allows a fluid communication between said product container (8) and said means for generating a flow (9), and between said means for generating a flow 9) and said port (4a) of said container (1a) and;
- wherein said redirection means (10) prevents a direct fluid communication between said product container (8) and said container (1a).

5. The device according to any one of claim **1 to 4,** wherein said device further comprises:
- a third container (1c),
wherein said container (1c) comprising at least a port (4c) and a port (5c);
- a second means for generating a flow (2b),
wherein said means is able to control the quantity of fluid to flow;
- a second redirection means (3b);
wherein said redirection means (3b) selectively allows a fluid communication between said port (5b) of said container (1b) and said means for generating a flow (2b), and between said means for generating a flow (2b) and said port (4c) of said container (1c);
and wherein said device does not comprise a direct fluid communication between said container (1b) and said container (1c).

6. The device according to claim **5,** wherein said redirection means (3b) is located between said container (1b) and said means for generating a flow (2b), and between said means for generating a flow (2b) and said container (1c).

7. The device according to claim **5** or claim **6,** wherein said container (1a) further comprises a port (6a), said container (1b) further comprises a port (6b), and said container (1c) further comprises a port (6c).

8. The device according to any one of claims **1 to 7,** wherein said redirection means are multi-way valves, preferably three-way valves, more preferably three-way stopcocks.

9. The device according to any one of claims **1 to 8,** wherein said means for generating a flow are syringes or fluid pumps.

10. A device according to any one of claims **1 to 9,** wherein said device is a sterile single-use device.

11. A device according to any one of claims **1 to 10,** wherein at least one of the containers of said device is prefilled with medium before use of the device.

12. Use of the device according to any one of claims **1 to 11** for preparing a desired concentration of a product to be infused.

13. The use according to claim **12,** wherein the product to be infused is a drug, a nutritional complement or a population of cells.

14. Use of the device according to any one of claims **1 to 11** for cell therapy, parenteral nutrition or drug administration.

15. The method for preparing a desired concentration of a product comprising the use of the device according to any one of claims **1 to 9,** wherein the containers of the device were prefilled with a desired volume of medium, the method comprising the following steps:
- a determined volume of the product is transferred to container (1a) through port (4a);
- the redirection means (3a) is configured so that port (5a) of container (1a) is connected to the means for generating a flow (2a);
- a determined volume of the mixture comprised in the container (1a) is transferred into the means for generating a flow (2a);
- the redirection means (3a) is configured so that the means for generating a flow (2a) is connected to port (4b) of container (1b);
- the content of the means for generating a flow (2a) is then transferred to the container (1b) through port (4b);
thereby the container (1b) contains a ready-to-be infused product at the desired concentration.

16. The method for preparing a desired concentration of a product comprising the use of the device according to any one of claims **5 to 9,** wherein the containers of the device were prefilled with a desired volume of medium, the method comprising the following steps:
- a determined volume of the product is transferred to container (1a) through port (4a);
- the redirection means (3a) is configured so that port (5a) of container (1a) is connected to the means for generating a flow (2a);
- a determined volume of the mixture comprised in the container (1a) is transferred into the means for generating a flow (2a);
- the redirection means (3a) is configured so that the means for generating a flow (2a) is connected to port (4b) of container (1b);
- the content of the means for generating a flow (2a) is then transferred to the container (1b) through port (4b);
- the redirection means (3b) is configured so that port (5b) of container (1b) is connected to the means for generating a flow (2b);
- a determined volume of the mixture comprised in the container (1b) is transferred into the means for generating a flow (2b);
- the redirection means (3b) is configured so that the means for generating a flow (2b) is connected to port (4c) of container (1c);
- the content of the means for generating a flow (2b) is then transferred to the container (1c) through port (4c);
thereby the container (1c) contains a ready-to-be infused product at the desired concentration.
